# EUROPÄISCHE PATENTSCHRIFT

(11) **EP 0 776 198 B1**
(45) Veröffentlichungstag und Bekanntmachung des Hinweises auf die Patenterteilung: **26.09.2001**
(21) Anmeldenummer: 95929105.5
(22) Anmeldetag: 12.08.1995
(51) Int. Cl.: A61K 9/70

(54) **TRANSDERMALES THERAPEUTISCHES SYSTEM MIT HYDROLYSESCHUTZ**
TRANSDERMAL THERAPEUTIC SYSTEM WITH PROTECTION AGAINST HYDROLYSIS
SYSTEME THERAPEUTIQUE TRANSDERMIQUE PROTEGE CONTRE L'HYDROLYSE

(30) Priorität: 20.08.1994 DE 4429663
(43) Veröffentlichungstag der Anmeldung: 04.06.1997
(73) Patentinhaber: LTS Lohmann Therapie-Systeme AG, 56626 Andernach (DE)
(72) Erfinder: FRANKE, Hanshermann, D-56566 Neuwied (DE); HORSTMANN, Michael, D-56569 Neuwied (DE)
(74) Vertreter: Flaccus, Rolf-Dieter, Dr.
(86) Internationale Anmeldenummer: EP9503204
(87) Internationale Veröffentlichungsnummer: WO9606600

(56) Entgegenhaltungen:
- EP-A- 0 272 562
- DE-A- 4 241 128

## Beschreibung

Bei der galenischen Umsetzung eines Arzneistoffs in eine Arzneiform ist die Stabilisierung von hydrolyseempfindlichen Arznei- oder Hilfsstoffen ein weitverbreitetes Anliegen. Unter Hydrolyse wird im folgenden die Spaltung einer Substanz durch den Einfluß von Wasser verstanden. Hierunter fallen im besonderen die hydrolytische Spaltung von Estern, Acetalen, Ketalen, Aminalen und die hydrolytische Spaltung von Peptidarzneistoffen.

Allgemein gilt, daß ein Schutz gegen Hydrolyse durch folgende Methoden erreicht werden kann:
1. Einstellen eines bestimmten pH-Wertes, bei dem sich experimentellen Befunden zufolge eine möglichst niedrige Geschwindigkeitskonstante für den Abbau des hydrolyseempfindlichen Bestandteils ergibt (1).
2. Komplexbindung des zu schützenden hydrolyseempfindlichen wirksamen Bestandteils durch Zusatz geeigneter, zur Komplexbildung befähigter Reaktionspartner (2). Dies führt immer dann zu einer Stabilisierung, wenn der hydrolytische Abbau ausschließlich aus dem nicht als Komplex vorliegenden, hydrolyseempfindlichen Bestandteil heraus erfolgt.
3. Verringerung der Löslichkeit des hydrolyseempfindlichen Bestandteils in einem Medium durch gezielten Zusatz geeigneter Substanzen (3, 4). Beispielhaft können hier die pH-Wert-Einstellung durch Pufferlösungen und die Bildung unlöslicher, nicht zu Solvolyse neigender Derivate genannt werden.
4. Entfernen von Wasser aus der Arzneiform oder weitgehendes Erhalten einer nahezu wasserfreien Umgebung des hydrolyseempfindlichen wirksamen Bestandteils in einer Arzneiform (5).

Sämtliche angeführten Lösungsansätze 1-4 haben in der pharmazeutischen Industrie eine sehr große Bedeutung und sind je nach Arzneiform mehr oder weniger verbreitet. Für die transdermale Applikation von Arzneistoffen durch sogenannte transdermale therapeutische Systeme sind die meisten oft nur bedingt geeignet. Die transdermale Verfügbarkeit eines Arzneistoffes richtet sich im wesentlichen nach seinen physiko-chemischen Eigenschaften. Diese werden durch pH-Veränderungen, Komplexbildungen und durch Derivatisierungen meistens ungünstig beeinflußt, weshalb die oben genannten Methoden 1-3 nur in bestimmten Fällen geeignet sind.

Die sicherste Methode ist daher, den in einem transdermalen therapeutischen System eingebetteten (gelöst bis dispergiert) wirksamen Bestandteil in einem weitgehend wasserfreien Milieu zu halten.

Der zu diesem Themenkomplex bekannte Stand der Technik kann dabei durch zwei methodische Ansätze beschrieben werden:
a) Die Herstellung einzeln dosierter Arzneiformen mit einem hydrolyseempfindlichen Arznei- oder Hilfsstoff erfolgt unter Ausschluß von Feuchtigkeit (Wasser oder Wasserdampf) bei Verwendung von Primär- und Sekundärverpackungsmaterialien, die für die in der Umgebung während der Lagerung vorhandene Feuchtigkeit eine Migrationsbarriere darstellen und hierdurch das Packungsinnere mit dem hydrolyseempfindlichen wirksamen Bestandteil weitgehend wasserfrei halten.
b) Die Verwendung von wasserbindenden Substanzen innerhalb der Sekundärverpackung, um die während der Lagerung eindringende Feuchtigkeit zu binden und um damit den Feuchtigkeitsgehalt innerhalb der Primärverpackung zu minimieren.
c) Die Verwendung von wasserbindenden Substanzen innerhalb der Primärverpackung, z.B. durch wasserbindende in den Gefäßdeckel integrierte Trockenmittel.

Im Gegensatz zu den meisten anderen Arzneiformen besitzen transdermale therapeutische Systeme eine extrem große Flächenausdehnung der Primärverpackung. Ferner gilt für pflasterförmige transdermale therapeutische Systeme, daß diese nicht geknickt oder gefaltet werden können, um die Verpakkung möglichst flächenarm zu gestalten. Damit stellt für solche Systeme die Primärverpackung eine entsprechend groβe Grenzfläche für die Migration von Feuchtigkeit in die Verpackung hinein zur Verfügung. Aus diesem Grund reichen die Methoden a) bis c) nicht immer aus, um den geforderten, maximalen Feuchtigkeitsgehalt auch über längere Zeiträume unter allen Lagerbedingungen sicherzustellen und um den hydrolyseempfindlichen wirksamen Bestandteil zu stabilisieren.
Ziel dieser Erfindung ist es daher, transdermale therapeutische Systeme, die einen hydrolyseempfindlichen wirksamen Bestandteil enthalten, dadurch zu stabilisieren, daß ein Eindringen von Feuchtigkeit in die einzeldosierte Arzneiform durch geeignetes Packungsmaterial weitgehend verhindert wird und dennoch einmal durch die Primärverpackung eingedrungene Feuchtigkeit nicht zur Hydrolyse des Bestandteils führt.

Dies wird erfindungsgemäß dadurch gelöst, daß die Arzneiform selbst einen wasserbindenden Bestandteil enthält. Durch den wasserbindenden Bestandteil der Arzneiform im transdermalen therapeutischen System wird durch die Primärverpackung eingedrungene Feuchtigkeit gebunden und führt nicht zur Hydrolyse des hydrolyseempfindlichen wirksamen Bestandteils im System. Dies gilt besonders für z.B. unter ungünstigen Lagerbedingungen durch die Primärverpackung eingedrungene Feuchtigkeit während der Lagerung.
Dabei ist die Art der Einarbeitung in die Arzneiform und der Zerteilungsgrad ohne Bedeutung für die Funktion der Feuchtigkeitsbindung zum Schutz vor Hydrolyse. Für die Verwendung einer Substanz als wasserbindender Bestandteil ist es ebenfalls unerheblich, in welchem Aggregationszustand sich dieses vor und nach der Einarbeitung befindet, solange die wasserbindende Funktion erhalten bleibt.

Bei einfachen halbfesten Zubereitungen, wie z.B. Salben, Pasten oder nicht-wäßrigen Gelen, kann dies durch Einarbeiten des wasserbindenden Bestandteils, z.B. durch Dispergieren in die flüssigen oder halbfesten Ausgangsstoffe, erreicht werden.

Bei pflasterförmigen, transdermalen Systemen kann der wasserbindende Bestandteil in das Wirkstoffreservoir selbst oder in andere vor oder hinter dem Wirkstoffreservoir befindliche Kleber- oder Reservoirschichten oder Steuermembranen eingearbeitet werden.

Da die Produktion von transdermalen therapeutischen Systemen, die einen hydrolyseempfindlichen Bestandteil enthalten, ohnehin unter weitgehendem Wasserausschluß erfolgt, kann die Einarbeitung der wasserbindenden Substanz in ein solches System sehr leicht in den Herstellungsprozeß integriert werden.

Weitere Ausgestaltungen der Erfindung sind entsprechend den Unteransprüchen vorgesehen. Dabei kann das System mehrere Schichten aufweisen und wenigstens eine Schicht einen wasserbindenden Bestandteil enthalten. Dabei kann der wasserbindende Bestandteil mineralischer oder nicht-mineralischer Natur sein.

Eine Ausgestaltung sieht weiter vor, daß die Matrix oder eine ihrer Schichten ein Acrylsäureestercopolymer, ein Polyisobutylen, ein Ethylen-Vinyl-Acetat-Polymer oder Styrol-Isopren-Blockpolymer oder ein synthetisches Isopren-Isobutylen-Copolymer oder einen Schmelzkleber enthält. Beispielsweise kann der Wirkstoff Acetylsalicylsäure sein und in der Matrix gelöst oder dispergiert vorliegen.
Ferner kann der Wirkstoff Bopindolol sein und in der Matrix gelöst oder dispergiert vorliegen.

Mit Vorteil beträgt der Anteil des wasserbindenden Bestandteils am gesamten Matrixmaterial mindestens 1 Gew.-%, bevorzugt bis 30 Gew-%..

Eine weitere Ausgestaltung der Erfindung sieht vor, daß der mineralische wasserbindende Bestandteil das Anhydrat eines Erdalkali- oder Alkalimetallsalzes ist.

Dabei kann der mineralische wasserbindende Bestandteil das Semihydrat oder das Anhydrat des Calciumsulfates sein.

Vorteilhaft ist es, wenn das Matrixmaterial den wasserbindenden Bestandteil in feindispergierter Suspension enthält.

Im übrigen kann der Wirkstoff als Dispersion eines wasserfreien Kristallisats vorliegen.

Eine weitere erfindungswesentliche Maßnahme sieht vor, daß das transdermale therapeutische System in einer Verpackung in gasdicht versiegeltem Packmittel angeordnet ist und das Packmittel ausreichend wasserdampfsperrend ausgerüstet ist, um die Wasserbindungsfähigkeit des wasserbindenden Zuschlagsstoffes über die Lagerdauer zu erhalten. Zusätzlich kann das Packmittel ein Trockenmittel enthalten.

Die Erfindung wird weiterhin anhand von Beispielen näher erläutert.

### Beispiel 1:

### Einzeldosiertes Acetylsalicylsäure-haltiges Oleogel.

Eine homogene Mischung von 26 g Acetylsalicylsäure (plv. subt.) und 10 g mikronisiertem Calciumsulfat werden unter Rühren in 26 g Miglyol® 812 dispergiert. Die entstandene Dispersion wird durch sukzessive Zugabe von bis zu 7 g hydrophober, kolloidaler Kieselsäure in eine ölige gelartige Dispersion überführt. Diese wird unmittelbar in geeignete Flachbeutel eindosiert, wobei der Flachbeutel anschließend gasdicht verschlossen wird.

### Beispiel 2:

### Acetylsalicylsäure-haltiges transdermales therapeutisches System

10 g Acetylsalicylsäure (plv. subt.) und 15 g mikronisiertes Calciumsulfat werden zu einer homogenen Pulvermischung verarbeitet, die anschließend unter Rühren in 200 g lösemittelhaltiges Polyacrylat eingearbeitet wird. Die erhaltene Suspension wird mit einem 300 µm Rakel auf eine silikonisierte Polyethylen-Folie ausgestrichen und die Lösemittel durch 20 minütiges Trocknen bei 80 °C entfernt. Der Klebefilm wird mit einer Polyesterfolie abgedeckt. Der so entstandene Verbund wird mit Hilfe von Schneidwerkzeugen auf die erforderliche Größe gebracht und in einer weitgehend wasserfreien Atmosphäre in das Primärpackmittel eingebracht und gasdicht verschweißt.

## Patentansprüche

1. Transdermales therapeutisches System (TTS) mit einem hydrolyseempfindlichen Wirkstoff in einer feuchtigkeitsminimierenden Verpacking und mit einem schichtartigen Aufbau einer feuchtigkeits- und wirkstoffundurchlässigen Rückschicht, einer den Wirkstoff enthaltenden Matrix und fallweise einer die Matrix abdeckenden Schutzschicht, **dadurch gekennzeichnet, daß** die Matrix einen die Wirkstofthydrolyse verhindernden wasserbindenden Bestandteil enthält.

2. Transdermales therapeutisches System nach Anspruch 1, **dadurch gekennzeichnet, daß** die Matrix mehrere Schichten aufweist und wenigstens eine Schicht einen, wasserbindenden Bestandteil enthält.

3. Transdermales therapeutisches System nach Anspruch 1, **dadurch gekennzeichnet, daß** der wasserbindende Bestandteil mineralischer Natur ist.

4. Transdermales therapeutisches System nach Anspruch 1, **dadurch gekennzeichnet, daß** der wasserbindende Bestandteil nicht-mineralischer Natur ist.

5. Transdermales therapeutisches System nach Anspruch 1 oder 2, **dadurch gekennzeichnet, daß** die Matrix oder eine ihrer Schichten ein Acrylsäureestercopolymer, ein Polyisobutylen, ein Ethylen-Vinyl-Acetat-Polymer oder Styrol-Isopren-Blockpolymer oder ein synthetisches Isopren-Isobutylen-Copolymer oder einen Schmelzkleber enthält.

6. Transdermales therapeutisches System nach einem oder mehreren der Ansprüche 1 bis 5, **dadurch gekennzeichnet, daß** der Wirkstoff Acetylsalicylsäure ist und in der Matrix gelöst oder dispergiert vorliegt.

7. Transdermales therapeutisches System nach Anspruch 1, **dadurch gekennzeichnet, daß** der Wirkstoff Bopindolol ist und in der Matrix gelöst oder dispergiert vorliegt.

8. Transdermales therapeutisches System nach einem oder mehreren der Ansprüche 1 bis 7, **dadurch gekennzeichnet, daß** der Anteil des wasserbindenden Bestandteils am gesamten Matrixmaterial mindestens 1 Gewichtsprozent, bevorzugt 5 bis 30 Gew.-% beträgt.

9. Transdermales therapeutisches System nach einem oder mehreren der Ansprüche 1 bis 8, **dadurch gekennzeichnet, daß** der mineralische wasserbindende Bestandteil das Anhydrat eines Erdalkali- oder Alkalimetallsalzes ist.

10. Transdermales therapeutisches System nach einem oder mehreren der Ansprüche 1 bis 8, **dadurch gekennzeichnet, daß** der mineralische wasserbindende Bestandteil das Semihydrat oder das Anhydrat des Calciumsulfates ist.

11. Transdermales therapeutisches System nach einem oder mehreren der Ansprüche 1 bis 10, **dadurch gekennzeichnet, daß** das Matrixmaterial den wasserbindenden Bestandteil in fein dispergierter Suspension enthält.

12. Transdermales therapeutisches System nach einem oder mehreren der Ansprüche 1 bis 11, **dadurch gekennzeichnet, daß** der Wirkstoff als Dispersion eines wasserfreien Kristallisats vorliegt.

13. Transdermales therapeutisches System nach einem oder mehreren der vorhergehenden Ansprüche, **dadurch gekennzeichnet, daß** es in Form einer halbfesten Zubereitung vorliegt.

14. Transdermales therapeutisches System nach einem oder mehreren der vorhergehenden Ansprüche, **dadurch gekennzeichnet, daß** es in Pflasterform vorliegt und eine geeignete Rückschicht, ein damit verbundenes Wirkstoffreservoir, bei Abwesenheit anderer Steuermechanismen eine die Abgabe des Wirkstoffs steuernde Membran, eine Haftklebeeinrichtung zur Befestigung des Systems auf der Haut und im Bedarfsfall eine vor der Applikation des Systems wieder ablösbare Schutzschicht umfaßt.

15. Transdermales therapeutisches System nach einem oder mehreren der Ansprüche 1-14, **dadurch gekennzeichnet, daß** es mindestens eine Polymermatrixschicht enthält.

16. Transdermales therapeutisches System nach Anspruch 15, **dadurch gekennzeichnet, daß** mindestens eine der Polymermatrixschichten ein Acrylsäurecopolymer enthält.

17. Transdermales therapeutisches System nach Anspruch 15, **dadurch gekennzeichnet, daß** das Wirkstoffreservoir in flüssigem oder halbfestem Aggregatzustand vorliegt.

18. Transdermales therapeutisches System nach Anspruch 15, **dadurch gekennzeichnet, daß** das Wirkstoffreservoir ein Oleogel enthält.

19. Transdermales therapeutisches System nach einem oder mehreren der Ansprüche 1 bis 18, **dadurch gekennzeichnet, daß** es in einer Verpackung in gasdicht versiegeltem Packmittel angeordnet ist und das Packmittel ausreichend wasserdampfsperrend ausgerüstet ist, um die Wasserbindungsfähigkeit des wasserbindenden Zusatzstoffes über die Lagerdauer zu erhalten.

20. Transdermales therapeutisches System nach Anspruch 19, **dadurch gekennzeichnet, daß** das Packmittel zusätzlich ein Trockenmittel enthält.

## Claims

1. Transdermal therapeutic system (TTS) comprising a hydrolysis-sensitive active substance in a moisture-minimizing pack, and having a layer-like structure comprising a backing layer impermeable to moisture and active substance, a matrix comprising the active substance, and optionally a protective layer covering the matrix, **characterised in that** the matrix comprises a water-binding component that prevents the hydrolysis of active substance.

2. The transdermal therapeutic system according to claim 1, **characterised in that** the matrix comprises several layers, and at least one layer contains a water-binding component.

3. The transdermal therapeutic system according to claim 1, **characterised in that** the water-binding component is of mineral nature.

4. The transdermal therapeutic system according to claim 1, **characterised in that** the water-binding component is of non-mineral nature.

5. The transdermal therapeutic system according to claim 1 or 2, **characterised in that** the matrix or one of its layers comprises an acrylic acid ester copolymer, a polyisobutylene, an ethylene-vinyl-acetate polymer, or styreneisoprene block polymer, or a synthetic isoprene-isobutylene copolymer, or a hot-melt adhesive.

6. The transdermal therapeutic system according to one or more of claims 1 to 5, **characterised in that** the active substance is acetylsalicylic acid and is present in the matrix in dissolved or dispersed form.

7. The transdermal therapeutic system according to claim 1, **characterised in that** the active substance is bopin-dolol, and is present in the matrix in dissolved or dispersed form.

8. The transdermal therapeutic system according to one or more of claims 1 to 7, **characterised in that** the water-binding component portion in the entire matrix material amounts to at least 1%-wt., preferably 5 to 30%-wt.

9. The transdermal therapeutic system according to one or more of claims 1 to 8, **characterised in that** the mineral water-binding component is the anhydrate of an earth alkaline metal salt or alkali metal salt.

10. The transdermal therapeutic system according to one or more of claims 1 to 8, **characterised in that** the mineral water-binding component is the semihydrate or the anhydrate of calcium sulfate.

11. The transdermal therapeutic system according to one or more of claims 1 to 10, **characterised in that** the matrix material contains the water-binding component in a finely dispersed suspension.

12. The transdermal therapeutic system according to one or more of claims 1 to 11, **characterised in that** the active substance is present as a dispersion of a water-free crystallizate.

13. The transdermal therapeutic system according to one or more of the preceding claims, **characterised in that** it is present in the form of a semi-solid preparation.

14. The transdermal therapeutic system according to one or more of the preceding claims, **characterised in that** it is present in the form of a patch and comprises a suitable backing layer, an active substance reservoir connected thereto, in the absence of other control mechanisms a membrane controlling the release of the active substance, a pressure-sensitive adhesive device for affixing the system on the skin, and, if required, a protective layer which is removable prior to application of the system.

15. The transdermal therapeutic system according to one or more of claims 1 to 14, **characterised in that** it comprises at least one polymer matrix layer.

16. The transdermal therapeutic system according to claim 15, **characterised in that** at least one of the polymer matrix layers comprises an acrylic acid copolymer.

17. The transdermal therapeutic system according to claim 15, **characterised in that** the active substance reservoir is present in liquid or semi-solid state of aggregation.

18. The transdermal therapeutic system according to claim 15, **characterised in that** the active substance reservoir contains an oleogel.

19. The transdermal therapeutic system according to one or more of claims 1 to 18, **characterised in that** it is present in a pack in a gas-tight sealed package or means of packaging, and that the package or means of packaging is rendered sufficiently water vapour-resistant to maintain the water-binding capacity of the water-binding additive over the storage period.

20. The transdermal therapeutic system according to claim 19, **characterised in that** the package or means of packaging additionally contains a drying agent.

## Revendications

1. Système thérapeutique transdermique (TTS) comprenant une substance active sensible à l'hydrolyse dans un conditionnement minimisant l'humidité et possédant une structure stratifiée d'une couche dorsale imperméable à l'humidité et à la substance active, d'une matrice contenant la substance active, le cas échéant, d'une couche de protection recouvrant la matrice, **caractérisé en ce que** la matrice contient un constituant qui fixe l'eau et qui empêche l'hydrolyse de la substance active.

2. Système thérapeutique transdermique selon la revendication 1, **caractérisé en ce que** la matrice présente plusieurs couches et au moins une couche contient un constituant qui fixe l'eau.

3. Système thérapeutique transdermique selon la revendication 1, **caractérisé en ce que** le constituant qui fixe l'eau est de nature minérale.

4. Système thérapeutique transdermique selon la revendication 1, **caractérisé en ce que** le constituant qui fixe l'eau est de nature non minérale.

5. Système thérapeutique transdermique selon la revendication 1 ou 2, **caractérisé en ce que** la matrice ou une de ses couches contient un copolymère d'ester acrylique, un polyisobutylène, un copolymère d'éthylène - acétate de vinyle ou un copolymère séquencé de styrène - isoprène ou encore un copolymère synthétique d'isoprène - isobutylène ou bien un adhésif thermofusible.

6. Système thérapeutique transdermique selon une ou plusieurs des revendications 1 à 5, **caractérisé en ce que** la substance active est de l'acide acétylsalicylique et est présente dans la matrice à l'état dissous ou à l'état dispersé.

7. Système thérapeutique transdermique selon la revendication 1, **caractérisé en ce que** la substance active est du Bopindolol et est présente dans la matrice à l'état dissous ou à l'état dispersé.

8. Système thérapeutique transdermique selon une ou plusieurs des revendications 1 à 7, **caractérisé en ce que** la fraction du constituant qui fixe l'eau, rapportée à la matière matricielle totale, s'élève à au moins 1 pour cent en poids, de préférence de 5 à 30 pour cent en poids.

9. Système thérapeutique transdermique selon une ou plusieurs des revendications 1 à 8, **caractérisé en ce que** le constituant minéral qui fixe l'eau est l'anhydrate d'un sel de métal alcalino-terreux ou de métal alcalin.

10. Système thérapeutique transdermique selon une ou plusieurs des revendications 1 à 8, **caractérisé en ce que** le constituant minéral qui fixe l'eau est le semi-hydrate ou l'anhydrate du sulfate de calcium.

11. Système thérapeutique transdermique selon une ou plusieurs des revendications 1 à 10, **caractérisé en ce que** la matière matricielle contient le constituant qui fixe l'eau sous la forme d'une suspension finement dispersée.

12. Système thérapeutique transdermique selon une ou plusieurs des revendications 1 à 11, **caractérisé en ce que** la substance active est présente sous la forme d'une dispersion d'un produit de cristallisation anhydre.

13. Système thérapeutique transdermique selon une ou plusieurs des revendications précédentes, **caractérisé en ce qu'**il est présent sous la forme d'une préparation semi-solide.

14. Système thérapeutique transdermique selon une ou plusieurs des revendications précédentes, **caractérisé en ce qu'**il est présent sous la forme d'un emplâtre et comprend une couche dorsale appropriée, un réservoir pour la substance active relié à la première citée, en l'absence d'autres mécanismes de commande, une membrane qui commande la distribution de la substance active, un mécanisme auto-adhésif pour la fixation du système sur la peau et, en cas de besoin, une couche de protection pelliculable avant l'application du système.

15. Système thérapeutique transdermique selon une ou plusieurs des revendications 1 à 14, **caractérisé en ce qu'**il contient au moins une couche matricielle polymère.

16. Système thérapeutique transdermique selon la revendication 15, **caractérisé en ce qu'**au moins une des couches matricielles polymères contient un copolymère d'acide acrylique.

17. Système thérapeutique transdermique selon la revendication 15, **caractérisé en ce que** le réservoir pour la substance active est présent à l'état liquide ou d'agrégat semi-solide.

18. Système thérapeutique transdermique selon la revendication 15, **caractérisé en ce que** le réservoir pour la substance active contient un oléogel.

19. Système thérapeutique transdermique selon une ou plusieurs des revendications 1 à 18, **caractérisé en ce qu'**il est disposé dans un conditionnement sous la forme d'un emballage hermétique étanche aux gaz et l'emballage est arrangé de façon à procurer un arrêt contre la vapeur d'eau suffisant pour conserver la capacité de fixation de l'eau de l'additif qui fixe l'eau sur toute la durée d'entreposage.

20. Système thérapeutique transdermique selon la revendication 19, **caractérisé en ce que** l'emballage contient en outre un dessiccateur.
